(19) Europäisches Patentamt
European Patent Office
Office européen des brevets

(11) Veröffentlichungsnummer: **0 339 501**
**A2**

(12) **EUROPÄISCHE PATENTANMELDUNG**

(21) Anmeldenummer: **89107187.0**

(22) Anmeldetag: **21.04.89**

(51) Int. Cl.⁴: **A61M 25/00**

(30) Priorität: **26.04.88 DE 8805477 U**

(43) Veröffentlichungstag der Anmeldung:
**02.11.89 Patentblatt 89/44**

(84) Benannte Vertragsstaaten:
**AT BE CH DE ES FR GB GR IT LI LU NL SE**

(71) Anmelder: **Muti, Arturo, Dr.**
**Wilsederweg 48**
**D-4000 Düsseldorf 31(DE)**

(72) Erfinder: **Muti, Arturo, Dr.**
**Wilsederweg 48**
**D-4000 Düsseldorf 31(DE)**

(74) Vertreter: **Hanewinkel, Lorenz, Dipl.-Phys.**
**Patentanwalt Ferrariweg 17a**
**D-4790 Paderborn(DE)**

(54) **Bronchial Untersuchungskatheter.**

(57) Untersuchungskatheter mit einem Schlauch (1), der einenends mit einem aufblasbaren Balg (2) umgeben ist und andernends einen Trichter (5) und ein Ventil (4) trägt, das über einen Verbindungskanal (3) mit dem Balg (2) verbunden ist.

Der Katheter ist für eine Einbringung in Brochialäste dimensioniert.

Fig.1

EP 0 339 501 A2

## Bronchial Untersuchungskatheter

Die Erfindung betrifft einen Untersuchungskatheter bestehend aus einem flexiblen Schlauch, dessen eines, mit einer Öffnung versehenes, unteres Ende ein aufblasbarer Balg umgibt, dessen Innenraum durch einen Verbindungskanal, der an oder in der Schlauchwandung verläuft, mit einem Ventil am anderen oberen Schlauchende verbunden ist, das einen sich erweiternden Einführungstrichter trägt.

Aus DE-PS 23 11 266 ist ein Beatmungskatheter, auch Endotrachealtubus genannt, bekannt, der zur Einführung durch den Mund in die Luftröhre geeignet ist und der der Durchführung von Beatmungsschläuchen dient. Seine Länge entspricht dem kurzen Weg bis zum Ende der Luftröhre, wo er durch Aufblasen des Balges, der dem Querschnitt der Luftröhre in ihrem unteren Bereich sich anpaßt, jeweils fixiert wird. Mit einem derartigen Tubus ist somit nur die Lunge als Ganzes zu erfassen und durch den Balg abzusperren. In der Lungendiagnostik und -therapie ist dieser Katheter deshalb nicht gebräuchlich.

Es ist Aufgabe der Erfindung, einen Untersuchungskatheter, der für die Bronchoskopie, Bronchografie und Bronchialtherapie geeignet ist, zu offenbaren.

Die Lösung der Aufgabe besteht darin, daß die Schlauchlänge entsprechend eines Einführungsweges durch eine Nase, einen Rachenraum, eine Luftröhre bis in einen der einzelnen Bronchialäste gestaltet ist und der Schlauch im zusammengezogenen Zustand einen Außendurchmesser aufweist, der etwas geringer als die Weite eines Bronchialastes ist und im geblähten Zustand den Bronchialast ausfüllen kann, daß der Schlauch aus hochflexiblem Polyurethanmaterial besteht und daß die Öffnung zentral am Schlauchende liegt.

Vorteilhafte Ausgestaltungen sind in den Unteransprüchen angegeben.

Der neuartige Bronchialkatheter ermöglicht es, ihn mit Hilfe einer strahlendichten Sonde unter Röntgenbeobahtung in jeden beliebigen der 19 Bronchialäste einzuführen und dort zu verankern. Danach kann ohne weitere Strahlenbelastung ein Bronchoskop, ein Greifer zur Probeentnahme oder ein Spül-oder Begasungsschlauch usw. ein- und ausgeführt werden. Die übrige Lunge bleibt bei einem solchen Eingriff unberührt und funktionsfähig. Das strahlenempfindliche teure Bronchoskop bleibt unbelastet. Diese Eingriffe können ohne die bei den konventionellen Methoden übliche Narkose ausgeführt werden.

Treten bei einer Probeentnahme oder aus anderen Gründen Blutungen auf, so bleibt der Katheter so lange abgesperrt, bis eine Stillung der Blutung oder eine Absaugung des Sekretes stattgefunden hat. Eine Belastung der übrigen Lunge tritt nicht auf. Ein späteres Aushusten des Blutes und Sekretes entfällt. Auch während eines Transportes eines Patienten mit Lungenblutungen kann der Bronchialkatheter vorteilhaft zur Absperrung des betroffenen Lungenlappens eingesetzt werden.

Der Schlauchquerschnitt entspricht zweckmäßig der international bekannten Maßeinheit "Charriere 14", jedoch sind je nach Bronchienquerschnitt auch Charriere 12, 13 oder 15 vorzusehen.

Der Schlauch ist zweckmäßig mit einem Kontrastmittel, z.B. Schwerspat, ausgerüstet, so daß er im Röntgenbild verfolgt werden kann.

Das Polyurethanmaterial hat den Vorteil hoher Oberflächenglätte und Elastizität, und es läßt sich mit Silikonöl, von dem es nicht angegriffen wird, als Gleithilfe beschichten, und ist leicht zu desinfizieren.

Als vorteilhaft hat sich eine Länge von 60 cm gezeigt.

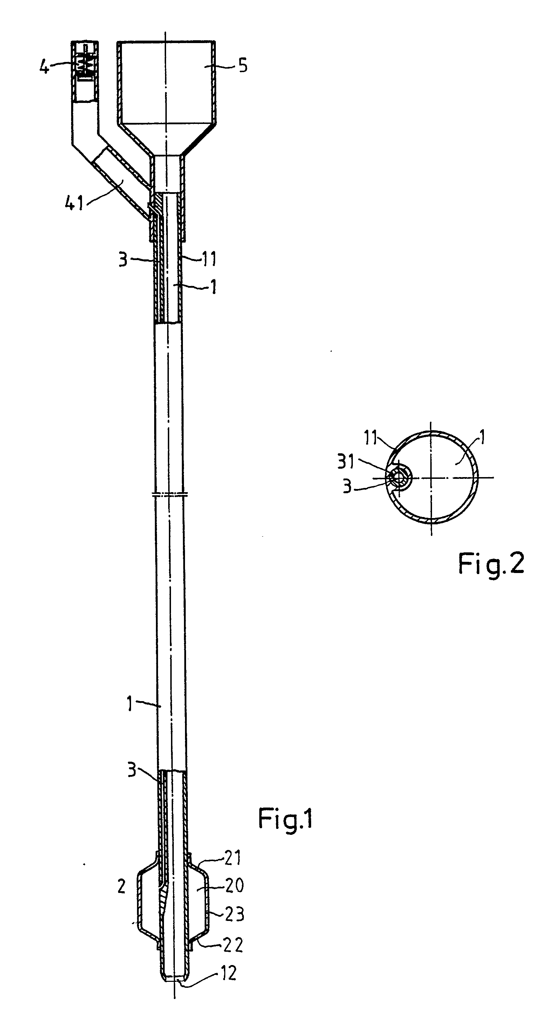

In den Figuren 1 und 2 ist eine erprobte Ausgestaltung dargestellt.

Fig. 1 zeigt vergrößert und verkürzt einen teilweisen Längsschnitt:

Fig. 2 zeigt einen Schlauchquerschnitt.

Fig. 1 zeigt am oberen Schlauchende einen Trichter (5) aus Kunststoff mit einem seitlichen Stutzen (41), das ein Ventil (4) enthält, das zur Lufteinführung und -absaugung mit einer Spritze geeignet ist. Der Stutzen (41) führt in den relativ engen Verbindungskanal (3), der andernends in den Balginnenraum (20) des Balges (2) führt, der nahe des unteren Schlauchendes, etwa mit 3 mm Abstand den Schlauch (1) umgibt. Der Schlauch (i) ist am unteren Ende leicht abgerundet gestaltet und weist eine zentrale Öffnung (12) auf. Der elastische Balg (2) hat eine solche Wandstärken-und Elastizitätsverteilung, daß er im aufgeblähten Zustand annähernd radiale Stirnwände (21, 22) und eine annähernd zylindrische glatte oder leicht gewellte Axialwand (23) aufweist.

Der Verbindungskanal (3) liegt als dünner Schlauch (31) in oder an der Schlauchwand (11), wie Fig. 2 zeigt, oder er ist unmittelbar in die Schlauchwand (11) eingeformt.

## Ansprüche

1. Untersuchungskatheter, bestehend aus einem flexiblen Schlauch (1), dessen eines, mit einer Öffnung (12) versehenes, unteres Ende ein aufblasbarer Balg (2) umgibt, dessen Innenraum (20) durch einen Verbindungskanal (3), der an oder in der Schlauchwandung (11) verläuft, mit einem Ventil (4) am anderen oberen Schlauchende verbunden ist, der einen sich erweiternden Einführungstrichter (5) trägt, dadurch gekennzeichnet, daß die Schlauchlänge gemäß einem Einführungsweg durch eine Nase, einen Rachenraum, eine Luftröhre bis in einen der einzelnen Bronchialäste gestaltet ist und daß der Balg (2) im zusammengezogenen Zustand einen Außendurchmesser aufweist, der etwas geringer als die Weite eines Bronchialastes ist, und im geblähten Zustand einen Bronchialast dichtend ausfüllen kann und daß der Schlauch (1) und der Balg (2) aus hochflexiblem Material bestehen und daß die Öffnung (12) zentral am Schlauchende liegt.

2. Untersuchungskatheter nach Anspruch 1, dadurch gekennzeichnet, daß die Schlauchlänge ca. 60 cm beträgt, der Schlauch (1) einen freien Innendurchmesser von 2,5 mm aufweist und der Balg (2) eine Länge von ca. 10 mm aufweist.

3. Untersuchungskatheter nach Anspruch 1 oder 2, dadurch gekennzeichnet, daß der Balg (2) etwa 3 mm vom unteren Schlauchende entfernt angeordnet ist und das Schlauchende abgerundet ist.

4. Untersuchungskatheter nach Anspruch 3, dadurch gekennzeichnet, daß der Balg (2) eine derartige Material- und Elastizitätsverteilung aufweist, daß er in dem geblähten Zustand annähernd radiale Stirnwände (21, 22) und eine annähernd zylindrische glatte oder leicht gewellte Axialwand (23) aufweist.

5. Untersuchungskatheter nach Anspruch 1, dadurch gekennzeichent, daß der Einführungstrichter (5) aus Kunststoff besteht und zentral am oberen Schlauchende befestigt ist und sich seitlich an ihm ein Stutzen (41) mit dem Ventil (4) befindet, der in den Verbindungskanal (3) führt.

6. Untersuchungskatheter nach Anspruch 1, dadurch gekennzeichnet, daß der Verbindungskanal (3) in die Schlauchwand (11) als relativ dünner Schlauch (3) eingelegt ist.

7. Untersuchungskatheter nach Anspruch 1, dadurch gekennzeichnet, daß der Verbindungskanal (3) unmittelbar in die Schlauchwand (11) eingeformt ist.

8. Untersuchungskatheter nach einem der vorstehenden Ansprüche, dadurch gekennzeichnet, daß sein Querschnitt Charriere 12 bis 15 entspricht.

9. Untersuchungskatheter nach Anspruch 8, dadurch gekennzeichnet, daß sein Querschnitt Charriere 14 entspricht.

10. Untersuchungskatheter nach einem der vorstehenden Ansprüche, dadurch gekennzeichnet, daß der Schlauch (1) und der Balg (2) aus Polyurethan und/oder aus Silikonkautschuk bestehen.

Fig.2

Fig.1